## Europäisches Patentamt
## European Patent Office
## Office européen des brevets

(19)

(11) Numéro de publication: **0 283 373**
**A1**

(12) **DEMANDE DE BREVET EUROPEEN**

(21) Numéro de dépôt: **88400519.0**

(22) Date de dépôt: **04.03.88**

(51) Int. Cl.⁴: **A 61 F 5/02**

(30) Priorité: **13.03.87 FR 8703485**

(43) Date de publication de la demande:
**21.09.88 Bulletin 88/38**

(84) Etats contractants désignés:
**AT BE CH DE ES GB GR IT LI LU NL SE**

(71) Demandeur: **SOCIETE DE FABRICATION DE MATERIEL ORTHOPEDIQUE**
**60-62 rue Rothschild**
**F-62600 Berk/Mer (FR)**

(72) Inventeur: **Cotrel, Yves**
**110 avenue Felix Faure**
**F-75015 Paris (FR)**

(74) Mandataire: **Martin, Jean-Paul et al**
**c/0 CABINET LAVOIX 2, Place d'Estienne d'Orves**
**F-75441 Paris Cedex 09 (FR)**

(54) **Vis vertébrale pour dispositif d'ostéosynthèse, notamment du rachis lombaire et dorsal.**

(57) Cette vis vertébrale (1) comprend une tige filetée (2) prolongée par un col profilé (3) et par un corps terminal (4), ces différentes portions étant de révolution autour de l'axe (X-X) de la vis; en coupe axiale, le col (3) présente, en combinaison, une partie concave (6) de largeur croissant dans la direction opposée à la tige filetée (2), puis une partie convexe (7) definissant la base du corps (4), le profil de la partie concave (6) étant déterminé pour permettre l'enfoncement de la vis (1) jusqu'au milieu (M) du col (3) environ, sans détérioration de l'élément osseux pédiculaire, tandis que la partie convexe (7) est dimensionnée de manière que le vissage ne soit pas gêné par les parties osseuses voisines.

FIG.1

EP 0 283 373 A1

# 0 283 373

## Description

### Vis vertébrale pour dispositif d'ostéosynthèse, notamment du rachis lombaire et dorsal

La présente invention a pour objet une vis vertébrale pour dispositif d'ostéosynthèse, notamment du rachis lombaire et dorsal, et du sacrum, comprenant une tige filetée prolongée par un col et par un corps terminal.

Dans les vis vertébrales connues, le col présente, en coupe axiale, une concavité partant de l'extrémité de la tige filetée et terminée par un angle vif délimitant la base du corps, lequel est percé d'une ouverture de passage d'une tige dimensionnée pour s'étendre le long de plusieurs vertèbres.

Ces vis doivent pouvoir être enfoncées au maximum dans le pédicule afin de pouvoir travailler en flexion. Or, on constate que l'enfoncement maximum susceptible d'être obtenu avec ces vis est insuffisant pour que leur travail en flexion soit réellement satisfaisant. De plus, le vissage et le coincement de la vis dans le pédicule soulèvent des problèmes, en raison notamment de l'encombrement du corps vis-à-vis des parties osseuses voisines (apophyses articulaires et transverses) qui peuvent gêner l'introduction de la vis et être éventuellement détériorées par le corps de cette dernière.

L'invention a donc pour but de remédier à ces inconvénients.

Suivant l'invention, le col de la vis présente, en coupe axiale, en combinaison une partie concave de largeur croissant à partir de l'extrémité de la tige filetée, puis une partie convexe définissant la base du corps, le profil de la partie concave étant déterminé pour permettre l'enfoncement de la vis jusqu'au milieu du col environ sans détérioration de l'élément osseux tandis que la partie convexe est di mensionnée de manière que le visage ne soit pas gêné par les parties osseuses voisines.

Suivant un mode de réalisation avantageux de l'invention, la partie concave est dimensionnée pour que le diamètre au milieu du col ne dépasse pas de plus de 60% environ le diamètre extérieur de la partie filetée.

L'enfoncement de la vis dans le pédicule peut ainsi être sensiblement supérieur à celui des vis connues, grâce à la géométrie particulière du col, ce qui permet un meilleur travail en flexion de la vis.

D'autres particularités et avantages de l'invention apparaitront au cours de la description qui va suivre, faite en référence aux dessins annexés qui en illustrent plusieurs formes de réalisation à titre d'exemples non limitatifs :

- la Figure 1 est une vue en élévation longitudinale partielle à échelle agrandie d'une vis vertébrale conforme à l'invention;
- les Figures 2, 3 et 4 sont des vues en élévation longitudinale de trois variantes d'exécution de la vis de la Figure 1;
- la Figure 5 est une vue en élévation longitudinale à échelle agrandie d'une vis selon l'invention enfoncée dans le pédicule d'une vertèbre et traversée par une tige métallique;
- la Figure 6 est une vue en élévation de deux vis enfoncées dans deux vertèbres correspondantes et traversées par une tige commune;
- la Figure 7 est une vue en élévation longitudinale d'un montage d'essai d'une vis conforme à l'invention traversée par une tige bloquée dans le corps de la vis par deux boulons;
- la Figure 8 illustre un montage d'essai pour l'étude de la résistance à la flexion d'une vis conforme à l'invention;
- les Figures 9A et 9B sont des vues en élévation longitudinale d'une vis selon l'invention dans deux positions d'enfoncement différentes;
- la Figure 10 est une vue en coupe transversale dans un plan coronal d'une vertèbre dorsale ou lombaire d'un dispositif d'ostéosynthèse équipé de vis selon l'invention.

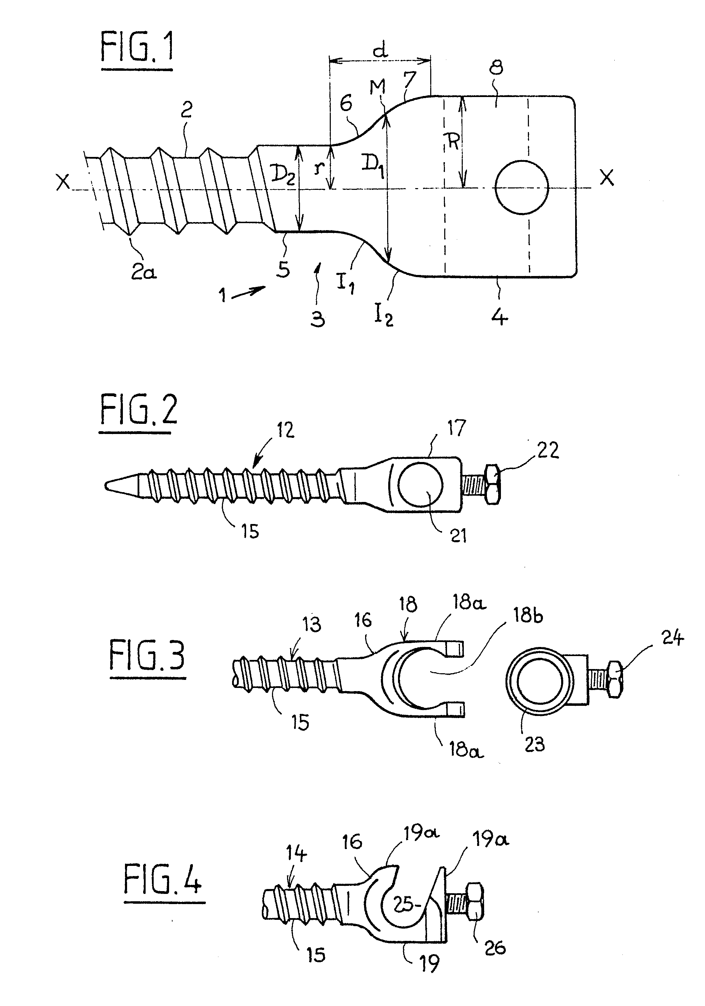

La vis vertébrale selon l'invention, représentée à la Figure 1 à échelle très agrandie, est destinée à faire partie d'un dispositif d'ostéosynthèse, notamment du rachis lombaire et dorsal ou du sacrum.

Cette vis 1, d'axe général X-X, comprend une tige filetée 2 dont l'extrémité, constituée par une partie cylindrique 5, est prolongée par un col profilé 3 et par un corps terminal 4.

Le col 3 s'étend sur une longueur d entre la partie cylindrique 5 et le corps 4 et présente les parties suivantes, en coupe axiale et à partir de l'extrémité cylindrique 5, dont le diamètre est égal à celui du sommet des filets 2a de la tige 2 :

a) une partie concave 6 dont le rayon r croît dans le direction opposée à celle de la tige filetée 2;

b) une partie convexe 7 dont l'extrémité, de rayon maximum R, définit la base du corps cylindrique 4.

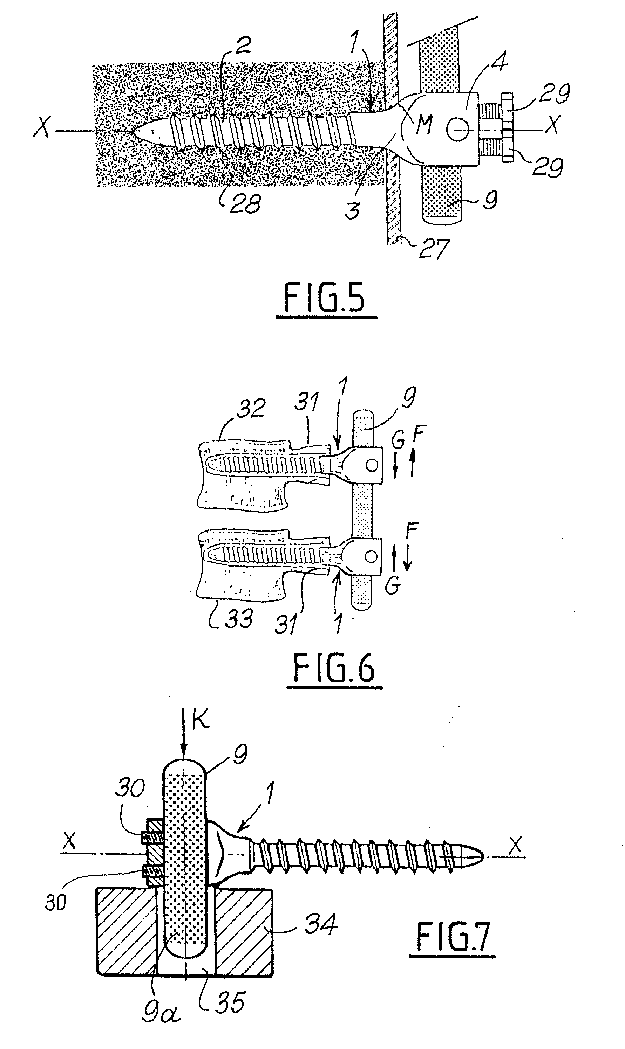

Ce dernier est percé de part en part, perpendiculairement à l'axe X-X, d'un alésage 8 destiné à recevoir une tige 9 (Figure 5).

Les parties concave 6 et convexe 7 présentent des points d'inflexion respectifs I1 et I2. Le profil de la partie concave 6 est déterminé pour permettre l'enfoncement de la vis 1 dans le pédicule d'une vertèbre lombaire ou dorsale jusqu'au milieu M environ du col sans détérioration de l'élément osseux, tandis que la partie convexe 7 et son rayon R sont dimensionnés de manière que le vissage ne soit pas gêné par les parties osseuses voisines (apophyses articulaires et apophyses transverses).

Suivant un mode de réalisation avantageux, la partie concave 6 est dimensionnée pour que le diametre D1 au milieu M du col (6, 7) ne dépasse pas de plus de 60% environ le diamètre D2 des sommets des filets 2a.

A titre d'exemple numérique indicatif, si D2=5mm, D1 ne doit pas dépasser 8 mm environ. En effet, une valeur de D1 sensiblement supérieure à la valeur précitée rendrait difficile l'introduction du col 3 dans l'élément osseux, et pourrait même détériorer celui-ci.

La géométrie particulière du col 3, constituée par la combinaison des parties concave 6 et convexe 7 avec les caractéristiques mentionnées ci-dessus, présente par ailleurs l'avantage de permettre d'enfoncer la vis au maximum dans le pédicule, et donc de lui permettre de travailler en flexion dans des conditions satisfaisantes, et ce sans provoquer de détérioration de l'élément osseux.

La vis 1 est réalisée en un matériau biocompatible, par exemple un acier inoxydable austénitique.

Les Figures 2 à 4 illustrent trois modes de réalisation possibles de la vis vertébrale conforme à l'invention.

Ces trois vis 12, 13, 14 comprennent chacune une tige filetée 15 et un col 16 identiques et diffèrent par leurs corps respectifs 17, 18, 19. Le corps 17 (Figure 2) est fermé et percé d'un alésage 21 pour le passage de la tige, ainsi que d'un trou de plus petit diamètre que l'alésage 21, s'étendant axialement à la vis et perpendiculairement à l'alésage 21 pour recevoir un boulon de serrage 22.

Le corps 18 de la vis 13 (Figure 3), à ouverture postérieure, se présente sous la forme de deux flancs 18a s'étendant de part et d'autre de l'axe de la vis 13 dans la direction opposée à la tige filetée 15. L'ouverture 18b délimitée par les flancs 18a est adaptée pour recevoir une pièce intermédiaire 23 de montage pour l'immobilisation de la vis 13 sur une tige à l'aide de deux boulons de serrage 24 (un seul étant visible à la Fig. 3).

Le corps 19 de la vis 14 comporte une ouverture latérale 25 délimitée par deux flancs 19a de réception de la tige, dont le serrage est assuré par deux boulons 26 (dont un seul est visible).

La Figure 5 montre le montage d'une vis pédiculaire 1 dans l'élément osseux 27, 28 du pédicule jusqu'au plan médian du col 3 défini par les points M. Le serrage de la tige 9 est assuré par deux boulons 29 engagés dans des trous du corps 4 ménagés parallèlement à l'axe X-X de part et d'autre de celui-ci, la vis 1 étant du type à corps fermé 4 selon la Figure 2.

Comme on l'expliquera ci-après plus en détail en référence à la Figure 7, le double blocage de la tige 9 par deux boulons 29 renforce considérablement la solidité de la fixation vis 1-tige 9 par rapport à la fixation à un seul boulon utilisée jusqu'à présent.

La Figure 6 montre l'implantation de deux vis 1 reliées pour une tige 9 dans les pédicules 31 de deux vertèbres 32, 33 du rachis lombaire ou dorsal. Les vis 1 remplissent une fonction d'ancrage vertébral pour permettre l'expansion (également appelée "détraction") ou la compression des vertèbres entre elles ou par rapport au sacrum. Ces mouvements de "détraction" et de compression sont respectivement symbolisés par les flèches F et G. Les vis pédiculaires 1 doivent surtout pouvoir résister aux forces perpendiculaires à leur axe (F, G), alors que les vis à os doivent résister principalement aux forces axiales d'arrachement. Pour ces dernières, le filet de la vis est l'élément le plus important, tandis que pour les vis selon l'invention, le corps et le col sont les éléments essentiels. Il a été constaté qu'au-dessous d'un diamètre de 3,5 mm, le corps de la vis est insuffisant pour résister aux forces habituellement nécessaires à la correction des déviations rachidiennes. C'est pourquoi on utilise habituellement deux diamètres de vis vertébrale adulte :

a) un diamètre extérieur de la partie filetée de 5 mm avec une longueur de 25 ou 40 mm, un filet de 0,75 mm et une âme de 3,5 mm;

b) un diamètre extérieur de 6 mm pour la partie filetée, une longueur de 40 mm, un filet de 0,75 mm.

Avant d'insérer une vis pédiculaire, il est nécessaire d'apprécier le diamètre du pédicule, et de s'assurer qu'il permet le passage de la vis, la largeur du pédicule variant en effet suivant les niveaux, les individus et la pathologie. Les tests mécaniques ont montré que la résistance aux forces perpendiculaires à l'axe de la vis est considérablement accrue, si celui-ci est enfoncé jusqu'à la moitié M du col (Figure 5), par exemple sur une distance de 2,5 mm à partir du début de la partie concave 6. Le vissage peut donc se faire jusqu'à ce niveau, la courbure particulière du col selon l'invention facilitant cette pénétration.

La Figure 7 illustre un dispositif permettant de tester la résistance mécanique de la fixation entre la vis 1 et une tige moletée 9 lorsque celle-ci est assurée par deux vis 30 enfoncées dans le corps 4 de part et d'autre de l'axe X-X.

Le corps 4 repose sur un bloc-support 34 percé d'un puits 35 de réception de la tige 9, le bloc-support 34 étant rigide afin d'éviter tout effet parasite de flexion. La résistance de la fixation à une force K exercée suivant l'axe de la tige 9 non perpendiculaire à l'axe X-X, est mesurée après vissage des vis 30 jusqu'à la rupture, la force K étant appliquée de façon progressive. Les résultats, comparés à ceux obtenus lorsque la fixation vis 1-tige 9 est assurée par une seule vis, ont montré que la fixation à deux vis 30 peut supporter un effort appliqué sur la tige 9 supérieur d'environ 35% à l'effort que peut supporter une fixation vis-tige réalisée par un seul boulon. A titre d'exemple indicatif, lorsque la fixation à un seul boulon résiste au maximum à un effort de 160 daN, la fixation à deux vis 30 peut résister à un effort de 210 daN.

Cette supériorité de la fixation à deux boulons provient de l'accroissement de stabilité des tiges filetées sur les pointes de diamant 9a de la tige moletée 9. L'effort ci-dessus de 210 daN est le seuil de glissement de la tige 9 par rapport au corps 4 de la vis.

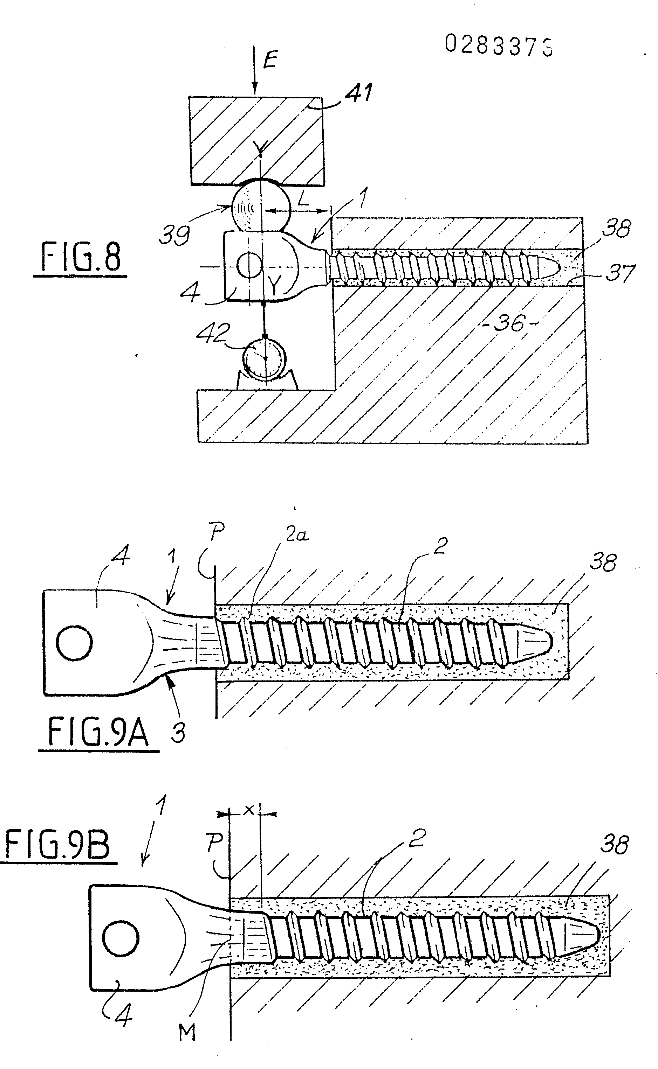

Le dispositif de la Figure 8 a pour fonction de permettre de tester la résistance de la vis 1 à la flexion en fonction de son degré d'enfoncement dans un socle rigide 36 de support. Dans celui-ci est ménagé un canal 37 rempli de résine époxy 38 permettant de simuler un ancrage de la vis 1 dans un os. Un effort progressif E est appliqué dans l'axe Y-Y de l'alésage du corps 4 par l'intermédiaire d'une bille 39 et d'une pièce 41. Afin de compenser les longueurs de bras de levier différentes dues aux conditions expérimentales, la distance entre l'axe Y-Y et le plan P d'encastrement géométrique (Figures 9A et 9B) a été mesurée à chaque essai, afin de corriger les résultats pour pouvoir les comparer. (Le plan d'encastrement réel est légèrement distant du plan d'encastrement géométrique en raison du module élastique de l'os).

Les valeurs de l'effort E appliquées à chaque essai ont été relevées pour une déformation radiale de la vis 1 de 0,5mm mesurée à l'aide d'un comparateur 42. Dans la position de la Figure 9A, la vis 1 n'est enfoncée que jusqu'à sa partie cylindrique 5, donc à l'exclusion du col 3, tandis que dans la position de la Figure 9B, un enfoncement supplémentaire x a été réalisé jusqu'au milieu M du col 3. Les deux tableaux ci-dessous contiennent les résultats numériques de trois essais exécutés dans chaque cas, avec des vis de diamètre D2 = 5mm et D2 = 6mm.

| vis | D2 = 5 mm | D2 = 6 mm |
|---|---|---|
| Bras de levier (mm) | 9,9 | 11,35 |
| Effort mesuré (daN) | 67 | 68 |
| Couple de flexion (mm.daN) | 663 | 772 |

Tableau 1 : résultats d'essais dans le cas d'encastrement de la Figure 9A

| vis | D2 = 5 mm | D2 = 6 mm |
|---|---|---|
| Bras de levier (mm) | 8,03 | 7,91 |
| Effort mesuré (daN) | 157,33 | 163 |
| Couple de flexion (mm.daN) | 1263 | 1289 |

Tableau 2 : résultats d'essais dans le cas d'encastrement de la Figure 9B

Les résultats de ces essais montrent que dans le cas de l'encastrement de la Figure 9A, la résistance à la flexion de la vis vertébrale d'un diamètre de sa partie filetée D2 = 6 mm est d'environ 16% supérieure à celle de la vis de diamètre D2 = 5 mm. Ceci peut s'expliquer par la différence des sections des vis au droit du plan P d'encastrement.

Par contre, dans le cas de la Figure 9B, où la vis est enfoncée d'une distance x = 2 mm supérieure à son enfoncement sur la Figure 9A, jusqu'au plan milieu M de son col 3, les résultats ne font plus apparaitre de différence notables de comportement entre les deux vis (tableau 2).

Il est possible d'interpréter ces résultats par le fait que la géométrie des deux vis au niveau du plan d'encastrement P dans le cas de la Figure 9B est sensiblement la même. En fonction de ces résultats, on peut conclure que l'effort supporté par la vis avant déformation est augmenté de 235% pour la vis vertébrale de 5 mm de diamètre et de 200% pour la vis de 6 mm de diamètre, entre un enfoncement au niveau de la fin du filet 2a de la vis (Figure 9A) et un enfoncement supplémentaire de x = 2 mm (Figure 9B) dans les conditions d'essais précités.

On peut également déduire de ces résultats les indications suivantes :

4

- il est nécessaire d'utiliser le double boulonnage pour la fixation vis-tige afin d'en augmenter l'efficacité;
- il est important que les vis pédiculaires soient vissées au moins jusqu'à la moitié de leur col 3, afin d'obtenir une excellente résistance à la flexion;
- la différence de résistance à la flexion entre une vis de 5 mm de diamètre et une vis de 5 mm de diamètre diminue en fonction du degré de l'encastrement, jusqu'à devenir inexistante.

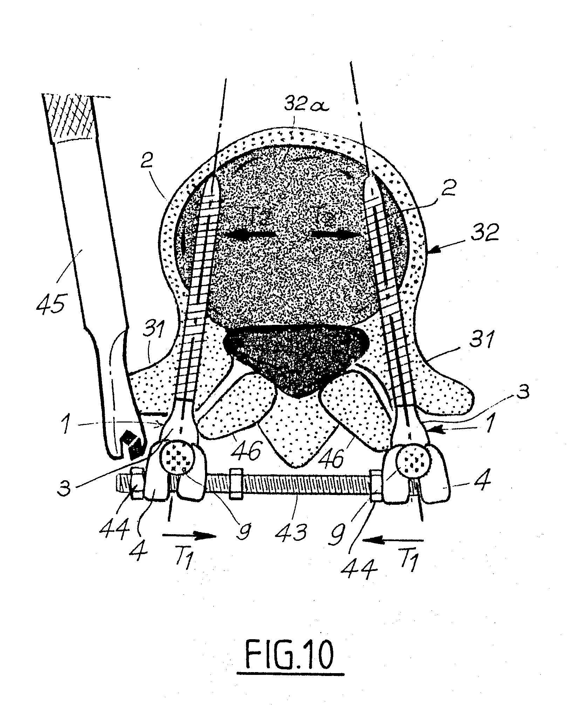

La Figure 10 montre un exemple de mise en oeuvre de vis vertébrale 1 selon l'invention dans un montage d'ostéosynthèse du rachis lombaire ou dorsal. Les vis 1 sont implantées à travers les deux pédicules 31 sur le même corps vertébral 32, convergent vers l'avant jusqu'à l'élément osseux 32a, sont traversées par deux tiges moletées 9 respectives et solidarisées entre elles en arrière par un dispositif de traction transversale à barrette filetée 43 traversant les corps 4. La barrette 43 est fixée en place au moyen d'écrous 44 serrés par un outil 45, en rapprochement en arrière des corps 4 et des deux vis 1.

Ce dispositif de traction transversale (appelé couramment "D.T.T.") assure, de part la géométrie du montage, une excellente résistance à l'arrachement. Du fait du rapprochement en arrière des corps 4 des deux vis 1 par ce dispositif "D.T.T.", les forces exercées par celui-ci étant symbolisées par les flèches T1, les parties filetées antérieures 2 des vis 1 tendent à venir au contact, sous l'effet des forces correspondantes T2 développées à ce niveau dans la vertèbre, avec la corticale latérale 32a du corps vertébral 32, plus résistante que l'os spongieux, ce qui améliore encore la stabilité du montage.

On remarque que les cols 3 et le corps 4 des deux vis 1 peuvent être mis en place jusqu'au degré d'enfoncement désiré dans les pédicules 31, sans que leur pénétration soit gêné par les apophyses 46.

Bien entendu, le montage de la Figure 10 n'est donnée qu'à titre d'exemple non limitatif parmi les différentes possibilités de mise en oeuvre des vis vertébrales selon l'invention dans des dispositifs d'ostéosynthèse.

## Revendications

1 - Vis vertébrale (1, 12, 13, 14) pour dispositif d'ostéosynthèse, notamment du rachis lombaire et dorsal, et du sacrum, comprenant une tige filetée (2) prolongée par un col profilé (3) et par un corps terminal (4), caractérisée en ce que, en coupe axiale, le col (3) présente, en combinaison, une partie concave (6) de largeur croissant dans la direction opposée à la tige filetée (2), puis une partie convexe (7) définissant la base du corps (4), le profil de la partie concave (6) étant déterminé pour permettre l'enfoncement de la vis (1) jusqu'au milieu (M) du col (3) environ sans détérioration de l'élément osseux, tandis que la partie convexe (7) est dimensionnée de manière que le vissage ne soit pas gêné par les parties osseuses voisines.

2 - Vis selon la revendication 1, caractérisée en ce que la partie concave (6) est dimensionnée de manière que le diamètre au milieu (M) du col (3) ne dépasse pas de plus de 60% environ le diamètre extérieur de la tige filetée (2).

3 - Vis selon l'une des revendications 1 et 2, dont le corps (4) est percé d'une ouverture transversale de passage d'une tige (9), caractérisée en ce que deux trous taraudés sont formés dans le corps (4) parallèlement à l'axe (X-X) de la vis (1) et de part et d'autre de celui-ci, afin de recevoir deux vis correspondantes (29 ; 30) de blocage de la tige (9).

4 - Vis selon l'une des revendications 1 à 3, caractérisée en ce qu'elle est constituée en un matériau biocompatible, par exemple un acier inoxydable austénitique.

0283373

FIG.1

FIG.2

FIG.3

FIG.4

0283373

FIG.5

FIG.6

FIG.7

0283373

FIG.8

FIG.9A

FIG.9B

0283373

FIG.10

## DOCUMENTS CONSIDERES COMME PERTINENTS

| Catégorie | Citation du document avec indication, en cas de besoin, des parties pertinentes | Revendication concernée | CLASSEMENT DE LA DEMANDE (Int. Cl.4) |
|---|---|---|---|
| X | FR-A-2 506 605  (M.B. ULRICH) <br> * Page 9, lignes 9-36; figures 4,5 * | 1 | A 61 F    5/02 |
| Y |  | 2,4 |  |
| Y | US-A-4 111 580  (J.G. FALCIONI) <br> * Figure 1; colonne 4, lignes 11-60 * | 2 |  |
| Y | AT-B-  293 458  (BÖHLER & CO.) <br> * Revendication 1 * | 4 |  |
| A | US-A-3 670 619  (W.M. COATS) <br> * Figure 6 * | 1,2 |  |
| A | FR-A-1 000 475  (E. URCELAY ASTEGUIA) <br> * Résumé; figures 1,4 * | 3 |  |
| A | FR-A-2 369 825  (A.L. LATYPOV et al.) <br> * Page 11, lignes 8-10; figures 1,3 * | 3 |  |
| A | DE-B-2 649 042  (M.B. ULRICH) <br> * Figures 1,2a,2b * | 1 |  |

**DOMAINES TECHNIQUES RECHERCHES (Int. Cl.4)**

A 61 F
A 61 B
F 16 B

Le présent rapport a été établi pour toutes les revendications

| Lieu de la recherche | Date d'achèvement de la recherche | Examinateur |
|---|---|---|
| LA HAYE | 09-06-1988 | WOLF C.H.S. |